# EUROPEAN PATENT SPECIFICATION

(11) **EP 1 499 373 B1**
(45) Date of publication and mention of the grant of the patent: **03.09.2008**
(21) Application number: 03722557.0
(22) Date of filing: 24.04.2003
(51) Int. Cl.: A61M 15/00

(54) **MEDICAMENT DISPENSER**
MEDIKAMENTENSPENDER
DISTRIBUTEUR DE MEDICAMENT

(30) Priority: 26.04.2002 GB 0209531
(43) Date of publication of application: 26.01.2005
(73) Proprietor: GLAXO GROUP LIMITED, Greenford, Middlesex UB6 ONN (GB)
(72) Inventor: JONES, Anthony, Patrick, Ware, Hertfordshire SG12 0DP (GB)
(74) Representative: Rice, Jason Neale
(86) International application number: PCT/EP2003/004402
(87) International publication number: WO 2003/090824

(56) References cited:
- EP-A- 0 689 848
- WO-A-02/36190
- DE-A- 19 748 725
- DE-A- 19 952 314
- US-A- 5 794 612

## Description

This invention relates to a medicament dispenser, and particularly to dry powder and metered dose inhalation devices, which have dose counters attached thereto. In particular, the invention relates to sensors used with dose counters to detect and validate release of medicament from the dispenser. When used herein, throughout the specification and claims, the terms "release of a medicament" and "medicament release" are used to refer to the release not only of a specific medicament, or combination of medicaments, but also to associated materials, such as carrier particles in the case of a powder dispenser and propellants in the case of an aerosol dispenser. These carrier particles or propellant materials are not medicaments of themselves, but they are contained in the release and may contribute significantly to the detection and validation process.

### Background to the Invention

Medicaments for treating respiratory disorders are frequently administered as dry powder formulations through the mouth and nose. Dry powder inhalation devices, or inhalers, are used in the administration of these drugs, inhalation by the patient resulting in uptake of a specified dosage of medicament through the nose or mouth.

The drug may be stored as a dry powder within a reservoir in the body of the inhaler, a metering chamber or other means being utilised to administer a specified dose of medicament. Alternatively, more sophisticated inhalation devices employ medicament carriers, such as individual capsules or blister packs/strips containing defined doses of powdered drug.

It is also known to use for such therapy medicaments which are contained in an aerosol and are administered to a patient by means of an inhalation device. The aerosol containers used in such inhalation devices are designed to deliver a predetermined dose of medicament upon each actuation and are known as Metered Dose Inhalers (MDIs); see Peter Byron, Respiratory Drug Delivery, CRC Press, Boca Raton, FL (1990) for a general background on this form of therapy.

Mechanical and electrical dose indicating devices, which typically count the number of doses delivered from or remaining in the medicament dispenser, are frequently used in dry powder inhalers and MDIs to enable patients to determine how much medicament is available in the dispenser for future use.

One problem associated with both mechanical and electrical dose counters is that they may, on occasions, give false readings. Thus the electronic dose counter disclosed in U.S. Patent Number 5,020,527 employs a mechanical trigger, which may be actuated without release of the medicament, thereby giving a false reading. Other forms of triggers are also known, particularly the use of sensing means to detect actuation of the dispenser.

U.S. Patent Number 5,544,647 discloses a range of sensors which may be used to detect and record actuation of MDIs. Typical sensors include pressure switches which are sensitive to either physical contact with a movable element of the device or to the inward breath of the patient. Other examples include sensors which are responsive to light, such as reflected or emitted light, or optical sensors which recognise reference points on the container or housing. These sensors are designed to detect movement of the aerosol container following actuation of the device by being aligned to a reference point or a light source, such as a LED. Sensors which are responsive to electromagnetic radiation, such as fluctuations in electromagnetic fields caused by movement of the container, are also disclosed within this document.

WO 95/07724 describes a dry powder inhaler with an electronic dose counter which employs a series of sensors to validate actuation of the inhaler. The inhaler uses magnetically responsive proximity reed switches to detect medicament loading within the device, and a thermistor sensor to detect temperature changes due to inhalation by the patient.

U.S. Patent Number 5,794,612 discloses a device for the administering with a propellant a dispensable medicament powder or liquid composition for inhalation by a patient which comprise a means for delivering aerosolized doses that includes an indicating system responsive to a selected range of ultrasound to display and/or record the dispensing of the powder and/or liquid dose. The indicating system comprises a means for sensing a selected range of ultrasound, a controller having a timer and which is associated with the sensor for signalling the sensed ultrasound, a means activated by said controller for displaying and/or recording each flow rate of propellant and a differential pressure sensor. Said document is considered to be the closest state of the art for the present invention, as it discloses all features of the preamble of claim 1.

A general problem encountered with dose counters in the art, such as those described above, is that they are dependent upon sensors which do not directly detect the medicament release from the dispenser. All of the sensors used in the art are arranged to sense some feature associated with actuation of the device, such as movement of the container/housing or pressure/temperature changes due to inhalation. This indirect method of sensing medicament release can lead to false readings being registered on the dose counter, if for example the sensor is activated without release of medicament. Accidental activation or triggering of the sensor may, for example, result from partial movement of the container or from interference with light received by the sensor. Similarly, a blockage or only partial release of the drug from either an MDI or dry powder inhaler would lead to a false reading registering on the dose counter.

Our co-pending International Patent Application No. PCT/EP01/12108 (published as No. WO 02/36190; and hereinafter referred to as "our said earlier application") discloses and claims a medicament dispenser provided with direct sensing means by which release of a medicament can be detected and recorded on a dose counter.

The invention disclosed in our said earlier application also comprises a means of confirming or validating medicament release from a medicament dispenser.

### Summary of Invention

The present invention relates to developed forms of the invention described in our said earlier application, which is hereby incorporated herein by reference in its entirety, and it relates especially to means for addressing practical operational issues such as contamination of the sensing means by any material included in the medicament release (whether of dry powder or aerosol form), or by condensation produced, for example, by moisture from a user's breath.

The invention also addresses further practical issues such as power saving and efficiency of operation.

### Detailed Description of Invention

According to one aspect of the present invention there is provided a medicament dispenser comprising:
(i) a housing having an outlet;
(ii) a medicament container locatable within said housing;
(iii) an electronic dose counter associated with said outlet; wherein said dose counter comprises a first sensor for directly detecting a medicament release dispensible from said medicament container through said outlet; and
(iv) means for detecting changes in the performance of the sensor attributable to contamination or degradation and for adjusting the operation of the dispenser to compensate, at least in part, for said changes.

Conveniently, the said means for detecting changes in the performance of the sensor comprises electronic means, utilising the sensor, for taking a calibration reading prior to the dispensing of a dose of medicament, comparing the calibration reading with a predetermined threshold value indicative of satisfactory operation of the sensor and for changing an operating characteristic of the sensor and/or of one or more electronic components associated therewith, in the event that the comparison indicates a change from said satisfactory operation, in a sense tending to restore satisfactory operation of the sensor.

Where the sensor includes an emitter and a detector of a beam of radiation, such as infra red radiation, the said operating characteristic preferably comprises the magnitude of driving current pulses applied to the emitter. In such circumstances, the calibration reading is preferably derived from electrical signals derived from the detector in response to radiation emitted by the emitter in response to said current pulses.

Further preferably, the magnitude of said current pulses is progressively increased from a starting level until said calibration reading matches said threshold level.

Instead of using a progressive increase of the said current pulses, they could, for example, be progressively decreased or varied in accordance with a technique of successive approximations or In accordance with another control regime and the invention in its broadest context is intended to include all such expedients, as well as that of progressive increase.

Preferably the said electronic means operates in accordance with a series of steps; allowing sensor performance to change step-wise, by predetermined threshold amounts prior to each restoratory change to the said operating characteristics. In such circumstances, the steps are preferably substantially equal in magnitude, but unequal steps may be used if desired.

Alternatively, the said electronic means may comprise part of a servo system that directly compensates for reductions in performance as they occur.

The first sensor preferably comprises an emitter, capable of emitting a beam of energy, and a detector capable of receiving and responding to energy in said beam.

The beam preferably comprises a collimated beam, but it may take any configuration determined by the requirements of the system and/or the geometry of a dispenser. For example, in some circumstances, it is preferred to have the beam conform to a flat, fanned shape and to provide a plurality of detectors, each responsive to the energy directed along a respective, finger-like portion of the fanned beam.

It is preferred in some circumstances to utilise reflective and/or refractive components or devices in order to "fold" the beam of energy, thereby to extend the region of interaction between the beam (or beams) of energy and the medicament release. In one example of such an arrangement, the emitter and a reflector are sited to opposite sides of a region through which a medicament release will pass when the dispenser is used: the arrangement being such that the beam of energy strikes the reflector and is reflected back through the region to a detector located to the same side of the region as the emitter. If desired, more reflectors can be provided; reflectors then being located to either side of the region, and being configured so that the beam undergoes successive reflections and crosses the region several times before being received by the detector, which may then, depending upon the number of reflections used, be located on the same side of the region as the detector, or on the opposite side. ,

In some embodiments, the reflector, or any or all of the reflectors in a multiple-reflector arrangement, my be replaced with or augmented by more complex optical components incorporating, for example, filters selective of the energy in the beam or beams utillsed and/or refractive components such as prisms Intended to divert unwanted energy away from the detector (or detectors).

The emitter suitably emits electromagnetic radiation and the detector detects the electromagnetic radiation.

The electromagnetic radiation emitted from the emitter may be radio frequency, infrared, visible or ultraviolet radiation. Suitably, radiation in the range 0.95µm to 0.35µm is used. More suitably, the radiation is in the infrared range. In particular, Infrared radiation with a wavelength of 0.88µm has been found to be useful.

In circumstances where the effects of ambient solar radiation could be troublesome, it may be preferred to operate at a wavelength which lies within known wavelength bands associated with significant absorption of solar radiation by the atmosphere, and at which the effects of solar radiation are thus much attenuated.

The emitter is suitably selected from the group consisting of light emitting diode, laser, incandescent lamp, electroluminescent and fluorescent light sources.

The emitter preferably emits Infra red radiation.

The emitter may include, or have associated therewith, a filter, suitably an optical filter and preferably a polarising filter (particularly if the emitter is an incandescent source) in order to select a particular wavelength or at least a narrow range or ranges of wavelength. Several advantages may be obtained by selecting a particular wavelength or range/ranges of wavelength, for example a given range of wavelengths may be especially sensitive to a particular drug/propellant combination. Alternatively, one 'sensitive' range and one 'insensitive' range may be selected - in this case the ratio of the two or more wavelengths reaching the detector would be used to detect the drug, thus making the sensor less prone to errors caused by an overall reduction in intensity due to contamination of the optical path.

The detector is suitably selected from the group consisting of photodiode, phototransistor, light-dependent resistor, pyroelectric and bolometer, and preferably it detects infra red radiation. In one embodiment, the detector additionally comprises a filter, suitably an optical filter and preferably a polarising filter. The-use of a filter will enable the wavelength/wavelengths detectable by the detector to be predetermined giving advantages similar to those described for using a filter with the emitter, for example, the detector could be made sensitive only to the wavelengths chosen for the emitter so the detector could be less sensitive to extraneous light source, such as room light/sun light. In a further embodiment, the detector is associated with an amplifier, since the output from the detector can be very small (of the order of micro Amps). Suitably, the amplifier is positioned as closed to the detector as possible to avoid amplifying any extraneous noise e.g. any electrical noise picked up in the connecting wires. In one particular embodiment, the amplifier is Integrated with the detector, for example the detector and amplifier are positioned on the same integrated circuit or "chip".

Where optical filters are used, they are typically employed to select specific wavelengths, whereas polarising filters select radiation of specific polarisation. It will be appreciated that polarising filters applied to both emitter and detector can conveniently be designed to select the same polarisation, though allowance has to be made in some circumstances for changes to the polarisation of emitted light by reflections and/or by interaction with the medicament release.

The sensor preferably comprises only a detector, for example a pyroelectric detector which responds to a decrease in temperature.

The detector may detect either an increase or decrease in radiation, compared to the amount of radiation emitted by the emitter. The increase or decrease may be due to interference of radiation reaching the detector by the medicament release.

The interference may be due to absorption, scattering, reflection, refraction or diffraction of radiation by the medicament release.

Typically, the interference results in a decrease in the amount of radiation reaching the detector, for example due to absorption, scattering, refraction or diffraction, resulting in a decrease In the output signal. Alternatively, the amount of radiation reaching the detector may be maintained at a substantially constant level by increasing the input level to the emitter. For example, an electronic feedback circuit that increases the current flowing through the emitter in order to maintain a substantially constant flux at the detector may be used, resulting in an increase in the current supplied to the emitter as the medicament is released.

Alternatively, the interference results in an increase in the amount of radiation reaching the detector, for example due to reflection by the medicament release, resulting in an Increase in the output signal. Alternatively, the amount of radiation reaching the detector may be maintained at a substantially constant level by decreasing the input level to the emitter. For example, an electronic feedback circuit that decreases the current flowing through the emitter in order to maintain a substantially constant flux at the detector may be used, resulting in a decrease in the current supplied to the emitter as the medicament is released.

If desired, the emitter, or a plurality of substantially co-sited emitters can be caused to emit radiation of more than one wavelength and the detector (or a plurality of detectors) may be selected to detect such radiation of more than one wavelength.

Preferably, the first sensor can quantify the concentration of medicament within the medicament release by measuring radiation at one or more wavelengths. These data can be processed, for example by a microprocessor, and compared against standardised data for a specified medicament to determine the concentration in the emission. For example, a first wavelength may be used as a control to calibrate the system response. Suitably, this wavelength is not affected by the medicament release. A second wavelength is affected by the medicament release, for example due to interference of the radiation by the medicament release. The ratio of the amount of radiation of the first wavelength to the amount of radiation of the second wavelength arriving at the detector will be dependent upon the concentration of medicament In the medicament release. If desired, a total dose can be quantified, for example by integrating the concentration signal.

The medicament dispenser may additionally comprise a second sensor (suitably having an emitter and a detector) for detecting a medicament release. Suitably, the second sensor is positioned such that the medicament release passes the second sensor subsequent to passing the first sensor. The presence of a second sensor may be used to increase confidence in the detection of the medicament release, for example for a detection to be considered valid, both sensors must be triggered. For example, a single sensor may be "triggered" by a foreign body interrupting the radiation path, but in this case the second sensor would not be "triggered"; thus the detection would not be considered valid and a dose not shown as given. Furthermore, the time lapse between triggering of the first sensor and triggering of the second sensor may be used to determine whether a detection is valid, i.e. the second sensor must be triggered within a specified time of the triggering of the first sensor.

The medicament dispenser may additional comprise a third sensor. Suitably, the third sensor is sensitive to parameters selected from the group consisting of electro magnetic radiation, electric field, magnetic field, light, motion, temperature, pressure, sound, oxygen concentration, carbon dioxide concentration and moisture. Preferably the third sensor responds to actuation of the dispenser.

Typically, the first and/or second sensor is integral with the outlet, for example moulded into the outlet, or is attached thereto. Alternatively, the first and/or second sensor may be reversibly attachable to the outlet and may be transferred from one outlet to another.

in the event that a third sensor is provided, it is typically integral with the housing, for example moulded into the housing, or attached thereto. Alternatively, the third sensor may be reversibly attachable to the housing.

The dose counter is associated electronically with the sensor(s), such that when the detector detects medicament release in the outlet, a signal is sent to the dose counter to record that a dose has been dispensed. The dose counter preferably comprises a microprocessor which performs operations on the data from the first sensor and produces a signal output relating to the data or to the outcome of an operation on the data.

In the event that second and possible also third sensors are utilised, the microprocessor preferably then performs operations on the data from the second or third sensor and produces a signal output relating to the data or to the outcome of an operation on the data. More preferably in such circumstances, the data from the second and/or third sensor is processed by the microprocessor to validate data from the first sensor.

The dose counter conveniently comprises a visual display unit for display of the data. Preferably, the visual display unit displays the number of doses of medicament used or remaining within the container. Preferably the doses are displayed numerically, by a series of coloured lights or by a monochrome bargraph.

Suitably, the dose counter is reversibly attachable to the housing.

In one embodiment, the first and/or second sensors are located on the dose counter (either integral therewith or detachable therefrom).

The medicament dispenser may additionally comprise one or more optical wave guides located within the housing. Suitably the one or more optical wave guide is composed of an organic polymeric or inorganic glass material in rod or fibre form.

Suitably, the medicament dispenser comprises two optical wave guides per sensor, i.e, one associated with the emitter and the other associated with the detector. A first optical wave guide channels radiation from the emitter to the outlet and a second optical wave guide channels radiation from the outlet to the detector.

Alternatively, the medicament dispenser comprises one optical wave guide per sensor, which may be associated with either the emitter or the detector.

Alternatively, the radiation emitted from the emitter may be reflected back to the detector from one or more reflective surface disposed on one or both sides of the outlet, thereby "folding" the beam of radiation in order to extend the area of interaction between the radiation and the medicament release. In such circumstances, defending on the geometry of the device and the number of reflections used, the emitter and detector may be located on the same side of the outlet. In which case the emitter and detector may be separate components or may be integrated into a single component. Any reflective surface thus employed may be provided by the surface of the outlet or by means of one or more additional components attached thereto. When the medicament dispenser is actuated, the medicament release interacts as described herein with the radiation of the beam, resulting in a discernible change in the signal received by the detector.

The sensor may conveniently be controlled by a digital or computational semiconductor device. Suitably, the digital or computational semiconductor device energise the sensor and associated electronic components to detect and respond to a medicament release every 0.1 to 100 ms. Preferably, the digital or computational semiconductor device energises the sensor and associated electronic components every 2 ms where the medicament release is in powdered form or every 40 ms for medicament releases of the aerosol type.

Conveniently, the sensor and associated electronic components are energised for 1 to 100 µs at each energisation. Preferably, the sensor and associated electronic components are energised for 20 µs where the medicament release is in powdered form or for 15 µs for medicament releases of the aerosol type.

For power saving purposes, it is preferred that the digital or computational semiconductor device be configured to return the sensor to low power mode after energising the sensor, either after a time-out period or after the detection of a medicament release. Preferably, the digital or computational semiconductor device returns to low power mode after the sensor has been de-energlsed.

The medicament container may, for example, comprise an aerosol container. Preferably, the aerosol container comprises a suspension of a medicament in a propellant; in one embodiment, the propellant comprises liquefied HFA134a, HFA-227, or carbon dioxide; in an alternative embodiment, the propellant comprises a mixture of one or more of liquefied HFA134a, HFA-227, or carbon dioxide. Alternatively, the aerosol container comprises a solution of a medicament in a solvent.

Alternatively, the medicament container may comprise a dry-powder container. Preferably the dry-powder container comprises medicament and optionally excipient in dry-powder form.

Medicament dispensers according to the invention may be actuated manually by the patient. Alternatively, the dispenser is actuated on application of mechanical or non-mechanical energy to a coupling element, for example one or more shape memory alloy (SMA) wires, for example a medicament dispenser as disclosed in WO 01/41849. Alternatively, the dispenser is actuated by the application of mechanical or non-mechanical energy to a drive means, for example as described in UK Patent Application No. 0114175.3.

The sensors of the present invention provide significant advantages for medicament dispensers which are actuated by the application of non-mechanical energy to a SMA coupling element, such advantages including: (i) preventing overheating of the SMA wires by shutting off the power as soon as they have contracted (heating the wires beyond this point does not result in any further contraction but may damage their microstructure), (ii) reducing or eliminating damage to the SMA wires by preventing the wires driving against a hard stop by switching off the power as soon as the drug is released, (iii) saving energy by reducing the SMA drive pulse to a minimum by shutting the power off as soon as the drug is released thus preventing the wires being heated for longer than necessary, and (iv) giving confirmation of device actuation.

For example, the control electronics could be configured to:
(i) switch off the drive to the SMA wires as soon as the sensor detects the drug. This would rely on the valve staying open long enough to allow complete release of the dose after power to wires was switched off;
(ii) start a timer at the start of the medicament release and then switch off the power to the SMA wires at a pre-set time thereafter;
(iii) wait until the sensor detects completion of medicament release and then switch off the power to the SMA wires;
(iv) wait until the sensor detects a particular point in the drug profile (such as the peak) and then switch off the power to the SMA wires immediately or after a pre-set time.

Preferably, the medicament is selected from the group consisting of albuterol, salmeterol, fluticasone propionate; beclomethasone dipropionate, salts or solvates thereof and any mixtures thereof.

Typically and conveniently, the outlet comprises a mouthpiece for inhalation therethrough.

The medicament dispenser may additionally comprise a communicator for communication to enable transfer of data from the dose counter to an electronic data management system. Preferably, data from the dose counter are transferable onto a local data management system.

In one example, the medicament dispenser additionally comprises a communicator for wireless communication with a gateway to a network computer system to enable transfer of data between the network computer system and the electronic data management system.

The present invention is intended to encompass the use of a medical dispenser according to any of the preceding claims to dispense medicament to a patient.

Energy may be conserved by a variety of means to enable the device to operate for longer on a given source of energy, such as a battery. Energy conservation or saving methods have additional advantages in terms of reducing the size requirements of the power source (e.g. battery) and thus the weight and portability of the inhalation device. In this respect, a variety of energy saving methods are available which generally involve reducing power consumption. One such method is to use a clock or timer circuit to switch the power on and off at regular or predetermined intervals. In another method the system can selectively switch on/off specific electronic devices, such as visual display units, electronic agitators or sensors, In order to power these devices only when they are required to perform a particular sequence of events. In particular, the components used for directly sensing medicament releases and for processing and displaying the sensed data, or data derived therefrom, may be energised in response to the detection of an event indicative that the medicament dispenser is about to be used, and may be switched off again after a predetermined time has elapsed, irrespective of whether the dispenser was actually used or not. Typically, such an event can comprise the manual or automatic advancing of a medicament strip in a powder dispenser, the opening of a mouthpiece, by displacement or removal of a cover, initial depression of an aerosol container or any other convenient metric, including the sensing of movement of or within the dispenser, such as those based upon sound, temperature, and pressure. Thus, for example, movement of an aerosol canister might be registered by interfering with a high frequency sound emanating from an emitter and sensed by an appropriate detector. Alternative detection methods may be used, based upon the emission and detection of a pressure wave (e.g. in the form of an acoustic wave) or of a heat wave.

A further alternative is to utilise a source-detector pair to detect movement within the inhalation device; typical examples including movement of the mouthpiece, loading, transport and preparation of medicament (e.g. removal of cover strip in a blister pack prior to medicament release) and sample metering (e.g. filling of metering chamber with medicament powder, aerosol or liquid).

Thus different electronic devices may be switched on and off atvarying intervals and for varying periods under control of the system.

Sensors, for example, may be energised at predetermined intervals (e.g. every 2 or every 40 milliseconds) and tor predetermined periods (e.g. 20 microseconds or 15 microseconds) thereby conserving power but being active long enough to detect specific stimuli, such as inhalation by the patient. Similarly, emitter-detector pairs may be controlled such that emitters are activated to pulse stimuli (e.g. electro magnetic radiation or sound) at predetermined intervals to appropriate detectors which are only energised to sense these stimuli at corresponding intervals. The power sequencing system may also respond to a sensor, such as a motion or breath sensor, which is activated on use of the device.

Low power or "micropower" components should be used within the electronics where possible and if a high power device is required for a particular function this should be put into a low power standby mode or switched off when not required. Similar considerations apply in the selection of transducers. Operation at low voltage is desirable since power dissipation generally increases with voltage.

For low power digital applications, complementary metal oxide semi-conductor (CMOS) devices are generally preferred and these may be specially selected by screening for low quiescent currents. Clock speeds of processors and other logic circuits should be reduced to the minimum required for computational throughput as power consumption increases with frequency. Supply voltages should also be kept at minimal values consistent with reliable operation because power dissipation in charging internal capacitance's during switching is proportional to the square of the voltage. Where possible, supply voltages should be approximately the same throughout the circuit to prevent current flowing through input protection circuits. Logic inputs should not be left floating and circuits should be arranged so that power consumption is minimised in the most usual logic output state. Slow logic transitions are undesirable because they can result in relatively large class-A currents flowing. Resistors may be incorporated in the power supply to individual devices in order to minimise current in the event of failure.

In some control applications, devices that switch between on and off states are preferred to those that allow analog (e.g. linear) control because less power is dissipated in low resistance on states and low current off states. Where linear components are used (e.g. certain types of voltage regulators) then types with low quiescent currents should be selected. In some circuit configurations it is preferable to use appropriate reactive components (i.e. inductors and capacitors) to reduce power dissipation in resistive components.

In order that the invention may be clearly understood and readily carried into effect, embodiments thereof will now be described, by way of example only, with reference to the accompanying drawings.

### Brief Description of the Drawings

Figure 1 shows a cross-sectional perspective of an MDI having a single sensor to detect medicament releases therefrom, and corresponds precisely to Figure 1 of our aforesaid earlier application;
Figure 2 shows a medicament dispenser and an associated system diagram;
Figure 3 shows a perspective view of a carrier for medicament in dry powdered form;
Figure 4 shows a base unit housing an internal mechanism for dispensing the medicament of Figure 3;
Figure 5 shows a base unit housing an alternative internal mechanism to that of Figure 4;
Figure 6a shows a perspective view of a dry powder medicament dispenser, in the form of a holder/body and a refill cassette, with the cassette removed from the holder/body;
Figures 6b and 6c show side views of the inner workings of the holder of the device of Figure 6a;
Figures 6d and 6e show side views of the Inner workings of the cassette of the device of Figure 6a;
Figure 7 shows, in simplified form, a base unit and replaceable cartridge with light guides to interconnect the optical paths;
Figure 8 shows a block diagram of electrical components used to electronically sense and compensate for contamination;
Figure 9 shows waveforms used to explain the operation of the arrangement of Figure 8;
Figure 10 shows schematically a baffle and vent arrangement to discourage and/or remove contamination;

### Detalled Description of Drawings

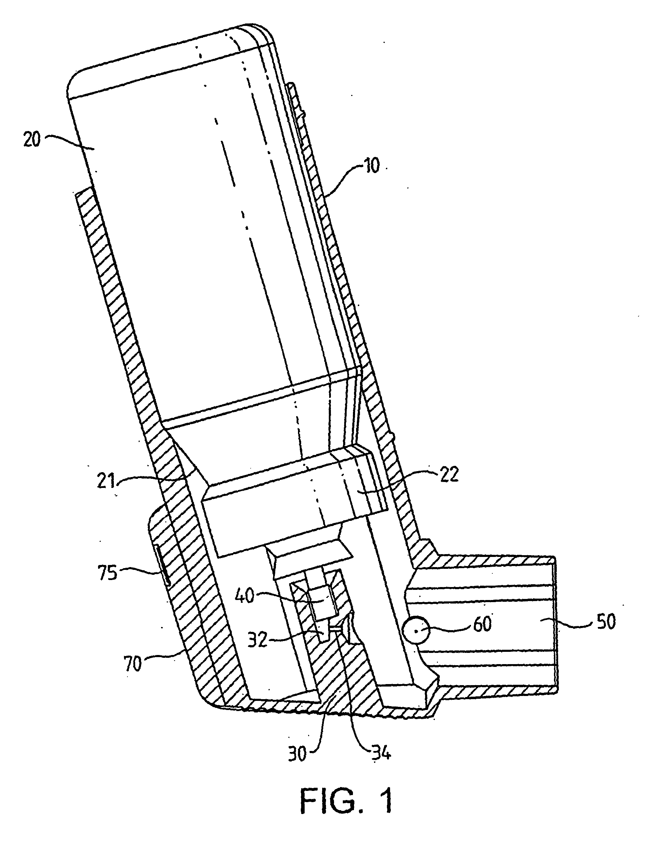

In our said earlier application, although it is made abundantly clear that the subject invention is applicable to dispensers of medicament in any suitable form including powders and aerosols for inhalation and also tablet form, most of the detailed description features aerosol-type dispensers. The present invention is also applicable to either type of dispenser, but much of the following detailed description will concentrate primarily upon dispensers intended for use with medicaments in powdered form. However Figure 1, now to be described, shows the aerosol container illustrated in and described with respect to Figure 1 of our aforesaid earlier application, for convenience and in order to emphasise to applicability of the present invention to either dispenser type.

Figure 1 depicts a sensor comprising an emitter/detector pair which respectively emit and are responsive to infra red radiation. It should be understood that other forms of sensors, such as those described hereinbefore, could equally be described in the drawings and used in the present invention. Furthermore, the sensor being described could alternatively comprise only a detector; thus lacking a corresponding emitter.

As shown in the cross-sectional perspective of Figure 1, an aerosol canister 20 is located in a housing 10 so that one end protrudes from the housing's open top; the canister being positioned such that its neck 21 and valve ferrule 22 are enclosed within housing 10. Spacer ribs (not shown) may be provided inside the housing to hold the external surface of the canister 20 spaced from the internal surface of the housing 10. A pilla-like support 30 is provided at the lower end of the housing 10 and has a passage 32 in which a valve stem 40 of the aerosol canister 20 can be located and supported. A second passage 34 is provided In the support 30 and is directed towards the interior of the outlet 50; the passage 34 communicating directly with both the passage 32 and the outlet 50. An emitter 60 is located on outlet 50. Emitter 60 emits a beam (not shown) of infra red radiation across outlet 50 onto a detector (not shown) attached to the other side of outlet 50. The emitter 60 may emit a continuous beam of infra red radiation or may emit a pulsed beam at, for example, intervals of 40 milliseconds and of duration 15 microseconds. The emitter 60 and the detector are operated under the control of a microprocessor within a dose counter unit 70. The detector (not shown) is responsive to changes in the amounts of infra red radiation detected thereby, and generates electrical signals, readable by the microprocessor, indicative of such changes. When the detected amount of energy in the infra red beam falls below a. predetermined threshold for a predetermined period (calibrated to respond to an emission of medicament released from canister 20) the microprocessor updates the display on a visual display unit 75 indicating the doses used or remaining within the canister.

The detector is calibrated to respond to an emission of medicament released from canister 20. The signal results in a change in the display on the visual display unit 75 indicating the doses used or remaining within the canister.

Thus, when the parts are in the positions shown In Figure 1, the protruding portion of the aerosol canister 20 can be depressed to move the canister relative to the valve stem 40 to open the valve and a dose of medicament contained in the canister 20 will be discharged as a medicament release through passage 34 and into the outlet 50 from which it can be inhaled by a patient. One dose will be released from the aerosol canister each time it is fully depressed. The medicament release will interfere with the beam of infra red radiation from emitter 60 resulting in a reduction in the amount radiation reaching the detector, compared with the amount received in the absence of a medicament release.

In this embodiment, the pulse length and repetition frequency imposed on the beam and the overall timing of the sensor's operation are such that several samples are taken before, during and after each medicament release. Thus not only can the reduction of infra red radiation received by the detector in the presence of a medicament release, as opposed to its absence, be directly sensed, but also the characteristic shape of the time profile of the reduction, as monitored at the detector, can be determined and utilised in the microprocessor to validate that a proper dosage of medicament has been released.

Instead of conducting microprocessor analysis of the time profile of the medicament release (commonly referred to as a "plume") alternative techniques, such as those described hereinbefore, can be used to validate a detected medicament release.

In any event, the visual display unit 75 is updated to display the number of doses used or remaining within the canister 20. This display may consist of a numerical read out, giving the precise number of doses used or remaining. Alternatively, the display may be generally indicative of the number of doses used or remaining, based upon a series of coloured lights (e.g. green, orange, red) representing an estimated range of the number of doses used or remaining.

Our said earlier application describes a number of further configurations, for example MDIs having two sensors to aid in validation of detected medicament releases, MDIs comprising detachable dose counter units, MDIs having rotatable outlets (mouthpieces) and MDIs wherein a refletive surface is used to direct infra red radiation from the emitter to the detector sited on the same side of an outlet as the emitter, thereby to achieve a "folded" path for the radiation which increases the size of the region of interaction between the infra red radiation beam and the medicament release and thus enhances the ability of the system to reliably detect medicament releases.

The present invention is applicable to all of the aforementioned configurations, but it is considered unnecessary to incorporate detailed descriptions of them herein.

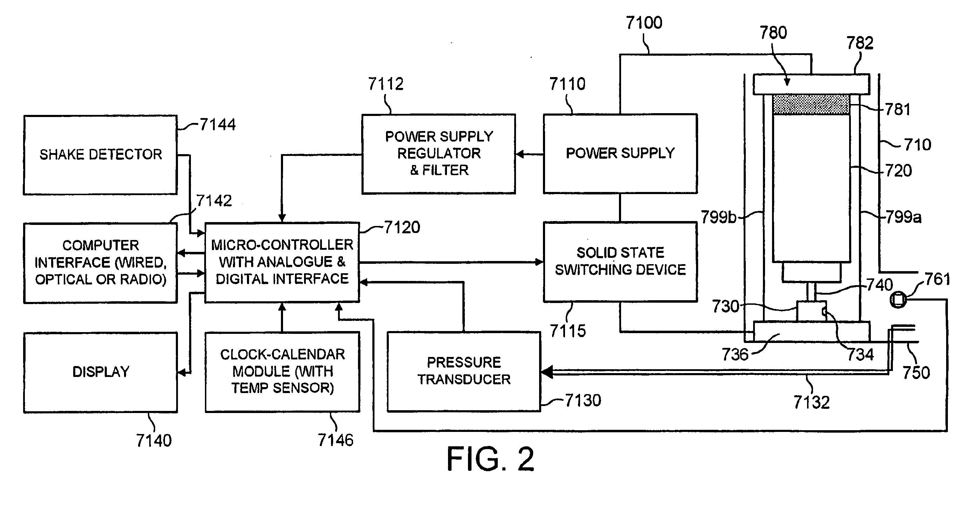

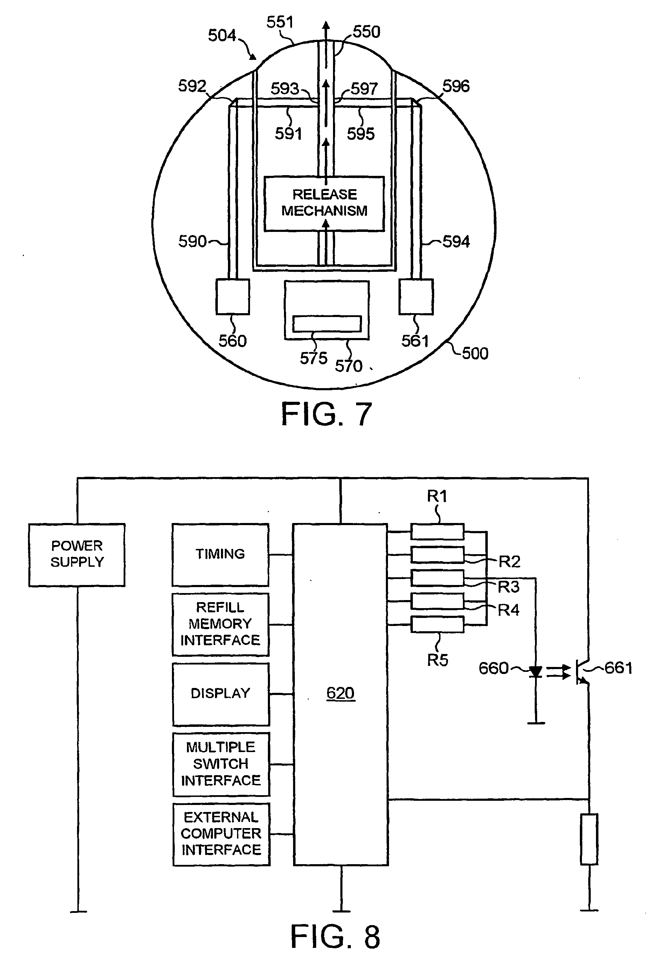

Figure 2 shows a schematic representation of a breath-operable medicament dispensing system and an associated system diagram, and corresponds to Figure 8 of our said earlier application.

The system illustrated in Figure 2 comprises a metered dose inhaler similar to that shown in more detail in Figure 1 comprising tubular housing 710 having a dispensing outlet 750 in the form of a mouthpiece. Within the housing 710 sits aerosol container 720 which has a valve. Valve stem 740 is supported by valve support 730. Outlet passage 734 is provided in the support 730 to enable passage of dispensed dose to the dispensing outlet 750. An emitter (not shown) emits a beam (not shown) of infra red radiation across outlet 750 on to a detector 761 attached to the other side of outlet 750.

It may be seen that the upper part of the aerosol container 720 abuts container seat 780. The container seat 780 comprises an insulating portion 781 which directly contacts the aerosol container 720 and an upper conducting portion 782 (e.g. comprised of aluminlum). It may also be seen that the valve support 730 connects with conducting valve seat 736. Plural shape memory alloy wires 799a, 799b connect the conducting portion 782 of the container seat 780 to the conducting valve seat 736. The plural wires 799a, 799b comprise a nickel-titanium alloy which contracts in response to electrical current flow therethrough. It may thus, be appreciated that on passage of electrical current through the plural wires 799a, 799b the container seat 780 and valve seat 736 will be drawn towards each other as the wires 799a, 799b contract. Actuation of the valve will result and a medicament release dispenser through outlet 750. The medicament release will interfere with the beam of infra red radiation from emitter (not shown) resulting in a reduction in radiation reaching the detector 761.

Control of electrical current flow to the container seat 780, valve seat 736 and wires 799a, 799b is achievable using the illustrated circuitry. Container seat 780 and valve seat 736 connect to actuation circuit 7100 which includes a power supply 7110 (e.g. a voltaic cell or battery of voltaic cells) and a switch 7115 in the form of a solid state switching device. The switch 7115 itself connects to control circuitry including micro-controller 7120 which has an analogue and digital interface. The power supply for the control circuitry is taken from the power supply 7110 for the wires 799a, 799b after suitable regulation and filtering 7112. The micro-controller 7120 itself connects with pressure transducer 7130 which has an input in the form of a pleasure tube 7132 located within the dispensing outlet 750 of the inhaler housing 710.

It may be appreciated that current flow to the container seat 780, valve seat 736 and wires 799a, 799b, and hence actuation of the dispenser may be achievable as follows. The patient inhales through the outlet 750 resulting in a change in pressure within the housing 710 and pressure tube 7132. The change in pressure is detected by the pressure transducer 7130 which sends a signal to the micro-controller 7120. The micro-controller 7120, in turn sends a switching signal to the solid state switching device 7115 which results in closing of the actuation circult and electrical current flow therethrough. The resulting contraction of the shape memory alloy wires 799a, 799b causes actuation of the dispenser and whence, dispensing of medicament to the inhaling patient. Interference of the infra red beam emitted by the emitter (not shown) is detected by the detector 761 and a signal sent to the micro-controller 7120 which can be configured to carry out one or more tasks. For example it may be configured to switch off the actuator circuit as soon as the medicament has been dispensed and to display an error message if the medicament is not dispensed.

It may also be seen in Figure 2 that the micro-controller 7120 is connected to a display 7140 for display of information to the patient and also with a computer interface 7142 for exchange of data therewith. Communication with the computer interface 7142 may be via a wired, optical or radio communications link. The micro-controller 7120 is also connected to shake detector 7144 for use in detecting whether the container 720 is shaken prior to actuation of the dispenser and to a clock-calendar module 7146 including a temperature sensor. All circuitry and components thereof including the power supply 7110, display 7140, shake detector 7144, computer interface 7142 and clock-calendar module 7146 may be arranged to be present on the housing 710 such that the system is in the form of a discrete, hand-held device.

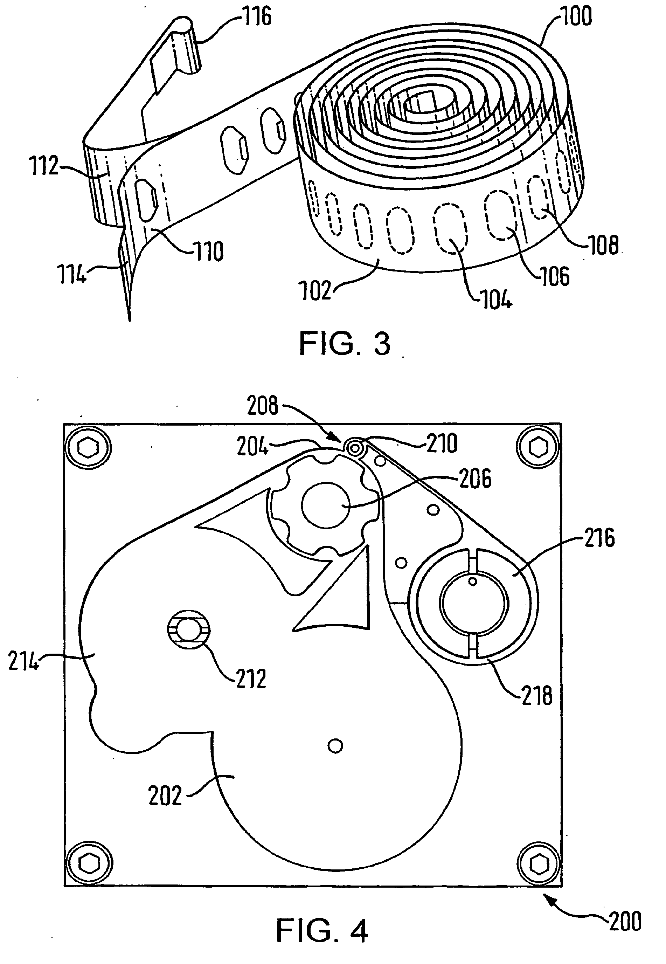

Figure 3 shows a medicament carrier 100 suitable for,use in a dry powder inhalation device, or inhaler, intended for use such that inhalation by the patient results in uptake of a specified dosage of medicament through the nose or mouth. The medicament carrier shown comprises a flexible strip 102 defining a plurality of pockets 104, 106, 108; each of which contains a dose of medicament, which can be inhaled, in the form of powder.

The strip comprises a base sheet 110 In which blisters are formed to define the pockets 104, 106, 108 and a lid sheet 112 which is hermetically sealed to the base sheet except in the region of the blisters in such a manner that the lid sheet 112 and the base sheet 110 can be peeled apart. The sheets 110, 112 are sealed to one another over their whole width except for the leading end portions 114, 116 where they are preferably not sealed to one another at all. The lid 112 and base 110 sheets are each preferably formed of a plastics/aluminium laminate and are preferably adhered to one another by heat sealing.

The strip 102 is shown as having elongate pockets 104, 106, 108 which run transversely with respect to the length of the strip 102 This is convenient in that it enables a large number of pockets 104, 106, 108 to be provided In a given strip 102 length. The strip 102 may, for example, be provided with sixty or one hundred pockets but it will be understood that the strip 102 may have any suitable number of pockets.

Figure 4 illustrates a base unit 200 of a medicament dispenser devised to dispense the medicament from strips such as 102. A medicament strip (not shown in this figure for clarity) is positioned in chamber 202 of the base unit 200. The strip is pre-fed through a guide member 204 within the manifold component and engaged in a six-pocket index wheel 206. The first pocket of the strip is positioned one pocket away from the opening station 208. The lid foil and base foil are separable about a beak 210. The resulting empty base foil is colled about a base take-up spindle 212 in the base take-up chamber 214. The used lid foil is fed over the beak 210 and coiled about a lid take up spindle 216 in the lid take-up chamber 218.

The dispenser is actuated by pressing a button on the side of the dispenser (not shown) which is linked to an electronic control system including a DC motor (not shown for clarity) to index the internal mechanism by one pocket of medicament The DC motor thus indexes the strip and coils up the waste foils.

Alternatively, a lever-operated manual dispensing procedure can be used, and is indeed preferred in some circumstances.

Initially, the gearing between the index wheel 206 and the lid take-up foil spindle 216 is one-to-one. However, as the lid take up spindle 216 winds on more foil, its effective winding diameter increases. An increase in diameter would cause the lid take-up spindle 216 to pull more strip than the index wheel 206 releases. Thus, in this particular example, the electronic control system is programmed to compensate for increase in lid spool diameter and adjust the amount the lid spool rotates accordingly.

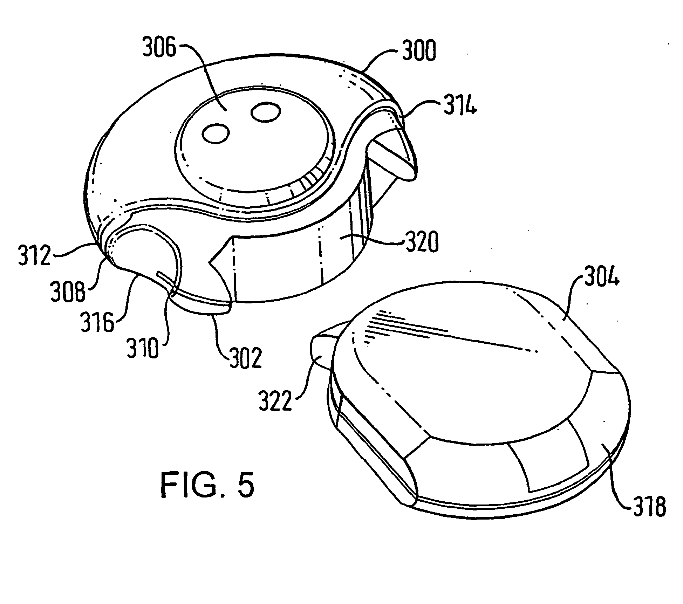

Figure 5 shows a medicament dispenser in external perspective view; the dispenser comprising a body 300, a holder 302, refill cassette 304 and electronic display 306. The holder 302 is shaped to fit snugly inside body 300 and is fixed to a point on the body (not shown) about which it rotates. Stops 308, 310 protrude from the holder 302 and prevent the holder 302 from rotating more than about 180° relative to the body 300. The stops 308, 310 also provide two defined positions of the holder 302 within the body 300. One position is defined by stop 308 meeting with body edge 312 and the other position defined by stop 310 meeting with body edge 314 when the holder has been rotated relative to the body. The area between stops 308 and 310 Is shaped to form a thumb or finger grip 316 for the user of the device. The holder 302 forms a shell into which the refill cassette 304 snugly fits.

The refill cassette 304 comprises a shell containing the medicament carrier (not shown) and a mechanism for opening the carrier (not shown) for the medicament to be accessed. The refill cassette 304 has a raised portion 318 at one end on both sides along its width so that this part of the refill cassette 304 is at least the same depth as the part of the holder 320 which receives the refill cassette 304. This allows the position of the cassette 304 within the holder 302 to be fixed such that the ridge 318 protrudes from the holder 302 but the rest of the cassette 304 is contained within the holder 302.

The refill cassette 304 also has a mouthpiece (not shown) and an actuating push button 322 for actuating the DC motor for Indexing the medicament carrier within the cassette 304.

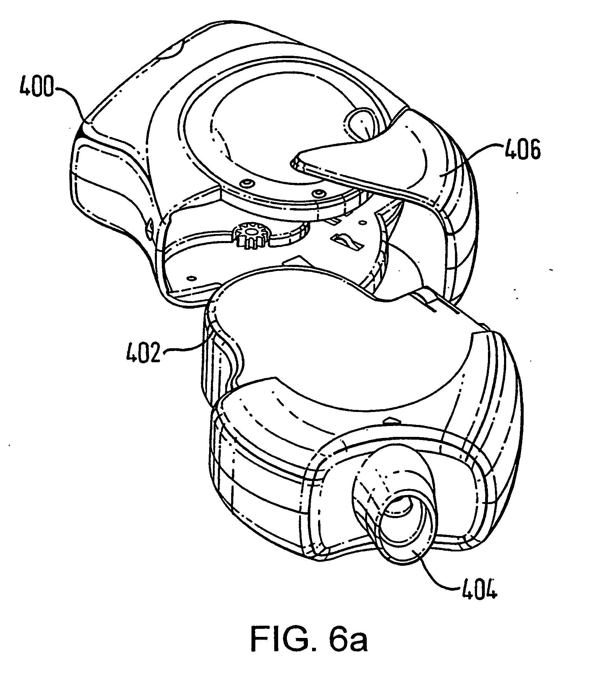

Figures 6a to 6e illustrate another example of a medicament dispenser with which the present invention can usefully be employed. The dispenser comprises a body 400 in the form of a cassette holder which receives a cassette 402. The cassette has a mouthpiece 404 which is covered by a rotating lid 406 when the cassette 402 is *in situ* in the body 400.

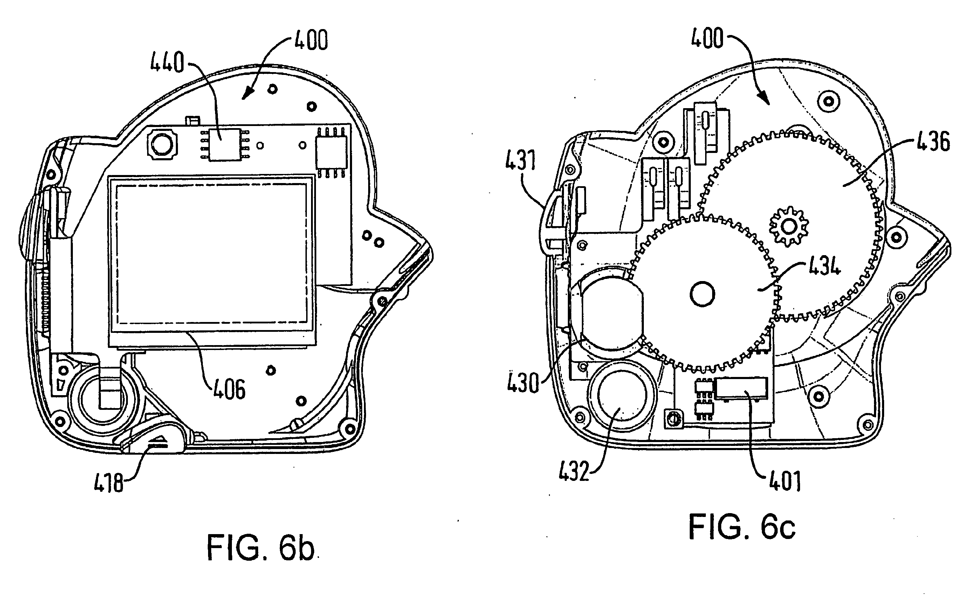

Figures 6b and 6c show a spilt-shell view of the holder 400 body shell. The body 400 is provided with DC motor 430 powered by battery 432, responsive to actuator switch 431. In use, the motor 430 drives gear wheels 434, 438, wherein gear wheel 436 drives foil spool 416 of the cassette 402 to advance a medicament dose. The body 400 is also provided with cassette release button 418 which releases a reversible catch mechanism (not shown) to enable mechanical release of a cassette 402 from the holder body 400. Electronic control system in the form of circuitry 440, 441 controls the DC motor 430, responsive to the actuator switch 431, and also controls LCD screen 406 for display of information to the user.

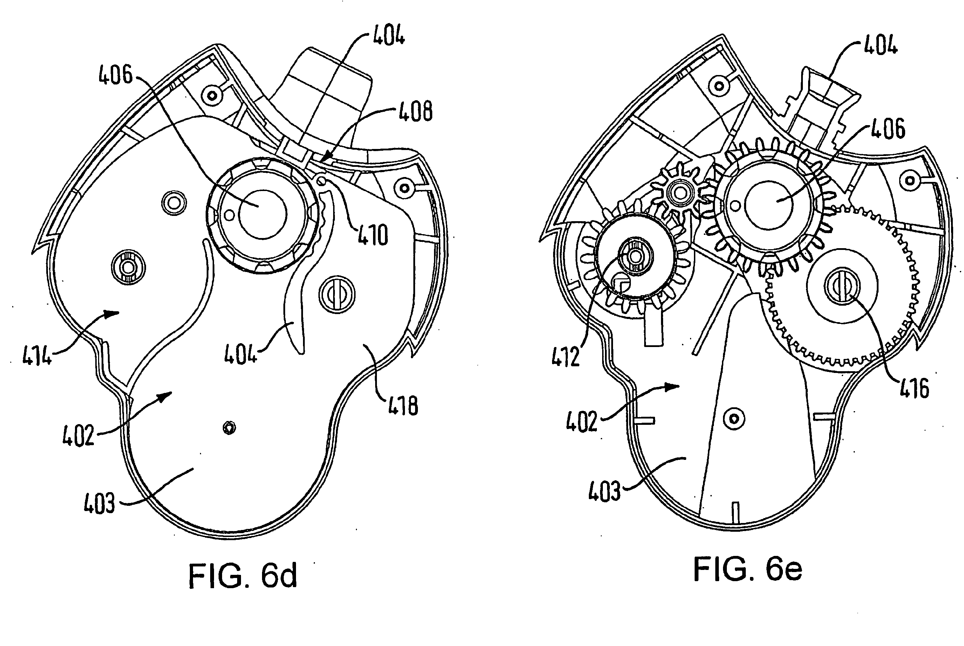

Figures 6d and 6e show the cassette 402 in more detail. A medicament strip (not shown for clarity) is portioned in chamber 403 of the cassette unit 402. The strip is pre-fed through a guide member 404 within the manifold component and engaged in a multi-pocket index wheel 406. The first pocket of the strip is positioned one pocket away from the opening station 408. The lid foil and base foil are separable about a beak 410. The resulting empty base foil is coiled about a base take-up spindle 412 in the base take-up chamber 414. The used lid foil is fed over the beak 410 and colled about a lid take-up spindle 416 in the lid take-up chamber 418.

The dispenser of Figures 6a to 6e is actuated by pressing the actuator button 431 on the side of the dispenser which is linked to the electronic control system 440, 441 which controls DC motor 430 to index the internal mechanism by one pocket of medicament. The DC motor 430 thus indexes the strip and coils up the waste foils.

Initially, the gearing between the index wheel 406 and the lid take-up foil spindle 416 is one-to-one. However, as the lid take up spindle 416 winds on more foil, its effective winding diameter increases. An increase in diameter would cause the lid take-up spindle 416 to pull more strip than the index wheel 406 releases. Thus, in this particular example, the electronic control system 440, 441 is programmed two compensate for an increase in lid spool 416 diameter and adjust the amount the lid spool 416 rotates accordingly.

The medicament dispensers described above are suitable for dispensing medicament, particularly for the treatment of respiratory disorders such as asthma and chronic obstructive pulmonary disease (COPD), bronchitis and chest infections. Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. as the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester), flunisolide, budesonide, rofleponide, mometasone e.g. as the furoate ester), ciclesonide, triamcinolone (e.g. as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothlazolone; adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetahydro-furan-3-4-diol (e.g. as maleate); α₄ integrin inhibitors e.g. (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g. as free acid or potassium salt), diuretics, e.g., amiloride; anticholinergics, e.g., ipratroplum (e.g. as bromide), tiotropium, atropine or oxitroplum; hormones, e.g., cortisone, hydrocortisone or prednisolone: xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) In combination with an anti-inflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

The medicament can take any suitable form including powder, aerosol and tablet from.

Generally, powdered medicament particles suitable for delivery to the bronchial or alveolar region of the lung have an aerodynamic diameter of less than 10 micrometers, preferably less than 6 micrometers. Other sized particles may be used if delivery to other portions of the respiratory tract is desired, such as the nasal cavity, mouth or throat. The medicament may be delivered as pure drug, but more appropriately, it is preferred that medicaments are delivered together with excipients (carriers) which are suitable for inhalation. Suitable excipients include organic exclpients such as polysaccharides (i.e. starch, cellulose and the like), lactose, glucose, mannitol, amino acids, and maltodextrins, and inorganic excipients such as calcium carbonate or sodium chloride. Lactose is a preferred excipient.

Particles of the powdered medicament and/or excipient may be produced by conventional techniques, for example by micronisation, milling or sleving. Additionally, medicament and/or excipient powders may be engineered with particular densities, size ranges, or characteristics. Particles may comprise active agents, surfactants, wall forming materials, or other components considered desirable by those of ordinary skill.

The excipient may be included with the medicament via well-known methods, such als by admixing, co-precipitating and the like. Blends of excipients and drugs are typically formulated to allow the precise metering and dispersion of the blend into doses. A standard blend, for example, contains 13000 micrograms lactose mixed with 50 micrograms drug, yielding an excipient to drug ratio of 260:1. Dosage blends with excipient to drug ratios of from 100:1 to 1:1 may be used. At very low ratios of excipient to drug, however, the drug dose reproducibility may become more variable.

It will be understood that the present disclosure is for the purpose of illustration only and the invention may be used with other forms of dispenser, notably dispensers in which the medicament is not held in strip form, as described above, but instead is held In a metering chamber, in individual dosage compartments or in a common compartment, to which access is rendered available by means of a moveable cover or the like.

For the sake of brevity in the following description, simplified drawings are used to depict generally dispensers of the kind shown in Figures 3 to 6, or alternative dispensers as discussed above, but omit all internal features that are not essential to the understanding of the invention. Moreover, the dispensers described with reference to the following drawings each incorporate a sensor comprising an emitter/detector pair which emit and are responsive to infra red radiation. It should be understood that other forms and/or numbers of sensors, as described hereinbefore, could equally be used in the present invention. Furthermore, the invention equally applies to dispensers having a sensor comprising only of a detector and lacking the corresponding emitter.

Each of the dry powder dispensers described in detail or otherwise referred to above can be fitted with components equivalent to those described in relation to the aerosol type dispensers described with reference to Figures 1 and 2 hereof, or otherwise mentioned in that connection, for directly sensing medicament release, for evaluating and/or validating a sensed release, and for providing an indication as to the number of doses dispensed from, and/or remaining within, the dispenser.

Whichever type of dispenser is employed, the present invention is concerned to address the potential for contaminants, deriving from the medicament release and/or induced or introduced by some external agency, to interfere with the sensing process, thereby to cause erroneous or spurious detection of medicament releases, failures of detection, or otherwise adversely influence the rellability of the sensing procedure and hence of the associated display of information to the patient

Typically, as will be understood from the foregoing, one or more beams of energy, usually in these examples infra red radiation, is transmitted at least once across an outlet near the mouthpiece of a medicament dispenser. This means in practice that there will be beam exit and entry areas, close to the mouthpiece itself, for the sensing beam which needs to interact with the medicament release. Surfaces disposed at these beam exit and entry areas are thus vulnerable to contamination by the medicament release or, for example, by condensation arising from the breath of a patient using the dispenser. Typically, such surfaces may comprise the actual surfaces of emitters and detectors; widows covering emitters and/or selectors; the exposed ends of light gulding components such as pipes or fibres; optical filtering or other optical processing components; reflectors, prisms and the like.

In any event, the present invention provides for the contamination to be addressed; various techniques being available for such purposes, depending to an extent upon the nature of the contamination anticipated, the construction and geometric layout of the dispenser, economy and other factors.

In particular, the invention envisages the provision of means for detecting changes in the performance of the sensor attributable to contamination and for adjusting the operation of the dispenser to compensate, at least in part, for such changes. Said means may be provided in combination with means for resisting deposition of contaminants at locations capable of adversely affecting the detection of said release by said sensor; and/or means for removing, at least in part, contaminants deposited at locations capable of adversely affecting the detection of said release by said sensor.

In Figures 7 et seq., features which are the same as those already described with reference to Figures 1 to 4 will not be further described unless they require to have certain characteristics in order to interface with the invention.

Referring now to Figure 7, an emitter 560 is mounted in a base unit 500 and can be energised (by means not shown) to generate the required radiation, in this case infra red radiation, for a beam of energy to be transmitted across an outlet 550, associated with a mouthpiece 551, incorporated in a replaceable cartridge 504, for detection by a detector 561 also mounted in the base unit 500. In order to couple the infra red radiation efficiently from the emitter 560 on the base unit 500, across the outlet 550 which Is part of the replaceable cartridge 504 and back to the base unit 500 for detection by the detector 561, light guides, such as optical fibres 590 and 594 respectively, are optically coupled to the emitter 560 and the detector 561. These light guides are caused to turn through respective right angles at 592 and 596 and are coupled respectively to an Input surface of a first relatively short light guide 591 and to an output surface of a second relatively short light guide 595. The output surface 593 of light guide 591 and the input surface 597 of light guide 595 are disposed, facing each other, to either side of the outlet 550. It will be appreciated that the patient is intended to suck on the mouthpiece in order to inhale the medicament.

Emitter 560 can thus emit an infra red beam (not shown) across outlet 550 onto the detector 561 by way of the light guides 590 and 591, across the outlet 550 and further by way of the light guides 595 and 594. It will be appreciated in this respect that the light guides 590 and 594 in the base unit 500 are disposed so as to align with the light guides 591 and 595 respectively In the cartridge 504 when the cartridge is correctly docked with the base unit, so that the infra red radiation is efficiently coupled between them. In some embodiments, an interlock is provided to inhibit medicament release unless the cartridge is properly docked with the base unit.

The emitter 560 may emit a continuous beam of infra red radiation, but it preferably emits a repetitively pulsed beam, typically comprising pulses of duration 20 microseconds emitted every 2 milliseconds, when the sensing procedure is operative. In this respect, for energy saving purposes, the emitter 560 may be energised so as to generate pulsed radiation as described only for a predetermined time following the occurrence of a recognisable event, such as the advancing of a strip of medicament pockets, as described hereinbefore, to expose a dose of medicament, and thereafter to generate a warning, such as illumination of a light emitting diode (LED), so as to indicate to the patient that a dose has been advanced and exposed but not taken, In these circumstances, in view of the risk that the dose will have been compromised in some way, such as by loss of some or all of the medicament directly through the outlet, or by absorption of environmental materials, the dispenser may be configured such that no further advancement of the medicament strip is possible until the patient has taken a clearing action which extinguishes the LED.

In any event, the emitter 560 is under the control of a microprocessor within a dose counter unit 570. The detector 561 is responsive to changes in the amounts of infra red radiation which it receives from time to time and generates an electrical signal readable by the microprocessor. When the level of energy falls below a predetermined threshold for a predetermined period (calibrated to respond to the release of a dosage of medicament from the dispenser) the microprocessor updates the display on a visual display unit 575 indicating the doses used and/or the number of doses remaining within the dispenser cartridge.

The detector is calibrated to respond directly to the release of an actual dosage of the medicament, and the signal thus results in a change in the display on the visual display unit 575 indicating the doses used or remaining within the cartridge.

The medicament release Interferes with the beam of infra red radiation from emitter 560, resulting usually, at least in the configuration currently being described, In a reduction in the amount of radiation reaching the detector 561. The visual display unit 575 is accordingly updated to display the number of doses used and/or remaining within the cartridge 504. This display may consist of a numerical read out giving the precise number of doses used or remaining. Alternatively, the display may merely be generally indicative of the number of doses used or remaining, based upon a series of coloured lights (e.g. green, orange, red) representing an estimated range of the number of doses used or remaining.

It will be appreciated from a consideration of figure 7 and its associated description that the beam of radiation that traverses the outlet 550 emerges from an output surface 593 of the light guide 591 to one side of the outlet 550, along which the medicament release passes, and enters the light guide 695 through an input surface 597. The surfaces 593 and 597 are disposed in such proximity to the medicament release and to the mouthpiece 551 that they are susceptible of contamination by the medicament release, and/or by external agencies such as condensation resulting from the breath of a patient.

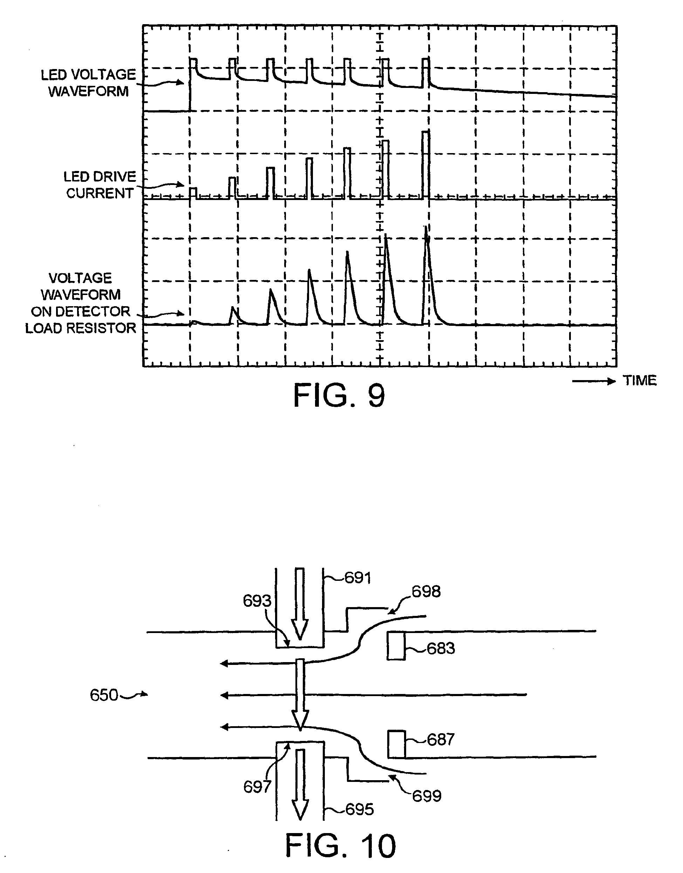

The present invention Is principally concerned with addressing the potentially deleterious attenuation of such contamination, such as attenuation of the infra red beam. Accordingly one embodiment of the invention, now to be described with reference to Figures 8 and 9, provides means for detecting changes in the performance of the sensor attributable to contamination and for adjusting the operation of the dispenser to compensate, at least in part, for such changes.

In the example of the invention shown with reference to Figures 8 and 9, the means for detecting change in the performance of the sensor comprises electronic means, utilising the sensor, for taking a calibration reading prior to the dispensing of a dose of medicament, comparing the calibration reading with a predetermined value Indicative of satisfactory operation of the sensor and for changing the operating characteristics of the sensor and/or of one or more electronic components associated therewith in the event that the comparison indicates a variance from said satisfactory operation in a sense tending to restore satisfactory sensor performance.

In particular, referring to Figure 8, the relationship between an emitter 660 and a detector 661 is monitored by a microprocessor 620, which also controls the operation of various other components associated with the sensor system and its display function. The microprocessor 620 controls the drive current applied to the emitter 660 by way of various resistive paths R1 to R5 and combinations thereof such that, when the microprocessor responds to a stimulus calling for the energisation of the emitter 660, it first applies a driving pulse of minimum operating current to the emitter 660, causing it to emit a pulse of infra red energy that traverses the outlet of the dispenser and impinges on the detector 661. The detector 661 produces an output signal, in response to its receipt of the pulse of infra red energy, comprising a current pulse which for convenience is converted into a voltage pulse which is received and analysed by the microprocessor 620.

The microprocessor compares the received voltage pulse with a threshold level indicative of a minimum level required for reliable operation. If the received voltage pulse exceeds the threshold level, the microprocessor continues to energise the emitter at the minimum current level. If, on the other hand, the received voltage pulse does not exceed the threshold level, the microprocessor selects an output configuration of the resistive paths R1 to R5 which permits the drive current applied to the emitter to be increased by a predetermined amount. The resulting voltage pulse received from the detector 661 is compared with the threshold In microprocessor 620 and, again, if the threshold is exceeded, the operation continues at that level whereas, If the threshold is not exceeded, the drive current used to energise the emitter is increased again; the process repeating until the drive current applied is sufficient to cause the detector to generate a voltage pulse that exceeds the threshold level.

It will thus be appreciated that the system always operates with the lowest current drive that is capable of providing reliable results, and that the system self-compensates for attenuation of the infra red beam attributable to contaminants deposited in the path of the beam, and especially upon surfaces in the optical path between the emitter 660 and the detector 661. Temporary contamination (such as condensation) will be compensated for only as long as it occurs, thus again leading to economies of power.

It will be appreciated that the foregoing procedure also compensates for ageing of electrical components.

The steps by which the drive current for the emitter 661 is increased may be equal or otherwise, depending upon choice, circuit configuration and operational criteria. In this respect, the procedure can usefully be set up to maintain operation of the detector 661 on a selected, sensitive part of its operating characteristic. The waveforms of Figure 9 show, by way of example only, a configuration utilising regular steps of equal size configured to keep the detector operating on a linear part of its characteristic.

Alternatively, the said electronic means may comprise part of a servo system that directly compensates for reductions in performance as they occur and thus, In an alternative procedure to that described with reference to Figures 8 and 9, the microprocessor 620 Is incorporated in a feedback loop that servos the emitter drive current to the detector output voltage so as to directly compensate for unwanted attenuations caused by contaminants.

It can be advantageous to provide for the microprocessor to activate a warning when it detects the need to apply, to the emitter 660, drive currents at or approaching its maximum capability, or that allowed for. By this means, severe attenuation of the beam caused for example by a long-term build up of, or an unfortunate combination of, contaminants can be recognised and the patient warned before the dispenser becomes unusable.

The microprocessor can also be configured to alert the patient or a supervisor, if the patient is a child or incapacitated for example, when a dose should be taken. This may be computed, for example, by elapsed time from the taking of the previous dose or, on the basis of a regular dosage regimen, at certain times of the day or night The alert may take any convenient form, such as an audible alarm such as a buzzer or bleeper; a flashing light or a vibratory signal (if the dispenser is likely to be carried on the patient's person), or any combination of the foregoing.

Preferably, the alert can be cancelled, either automatically in response to the detection of an event associated with the required dose being taken, or manually by the patient when the dose is taken, and times out after a prescribed period has elapsed without the alert being cancelled. In the latter event, it is useful for the dispenser to maintain a warning to the effect that a prescribed dose has been missed.

It can also be useful, for example in tracking compliance of the patient with a prescribed dosage regimen, to configure the microprocessor so that it records the pattern of dosage taking by the patient and to provided a docking connection whereby such information can be downloaded by or for a physician involved in treatment of the patient.

Instead of (or in addition to) merely recording and/or locally storing/displaying information locally at the medicament dispenser, facilities may be provided for transmitting any or all of the information to a remote location, for example a central processing station in a hospital or other clinical establishment Such transmission can be achieved either directly from the dispenser, via a suitable encoding and transmitting device, or Indirectly via another device, such as a sophisticated mobile telephone instrument, into which the required information is downloaded by way of a docking connection with the dispenser.

In embodiments of the invention in Which light guides are employed, it Is in some circumstances, where the use of optical components such as filters etc. is desirable, to provide all or some of such components at locations other than at the ends of the light guides. In particular, filter components may be provided at one or more locations intermediate the ends of a light guide to provide a distributed optical component. Moreover where, as in the arrangement of Figure 7 for example, a light guide is requires to undergo a significant angular deviation, such as a 90 degree turn, it may be preferred to utilise an optical component such as a prism at such a location and to take advantage or the refractive performance of the prism to improve the selectivity of the system with regard to a preferred wavelength, or range of wavelengths. Convenlently, in such an arrangement, the input and/or output surfaces of the prism may be coated to enhance the wavelength selection.

It has previously been mentioned that, for sensing the medicament release from a dry powder dispenser, it is preferable to operate the emitter in pulses, each of duration about 20 microseconds, repeated at intervals of 2 milliseconds. For an aerosol dispenser, the corresponding preferred values are 15 microseconds and 40 milliseconds. However, different values of these parameters may be required in various operational circumstances, and it is envisaged that pulse durations in the range from 1 to 100 microseconds could be used, repeated at intervals of 0.1 to 100 milliseconds. In some circumstances, it is advantageous for the pulse length and/or the repetition rate to be changed during operation, if an operations watchdog program operating within the microprocessor indicates that the sensor is operating at less than optimal efficiency in relation to a certain type of medicament release or in a dispenser of a certain geometry, for example.

Certain other expedients can usefully be employed, alone or in conjunction with any of the foregoing, to enhance the practical operation of dispensers of the various kinds described herein. For example, digital or analogue filtering can be applied to the detector output signals in order (inter alia) to reduce the effects of transient contamination, such as condensation. In this case, the filter characteristics are chosen to take advantage of the fact that the time constants associated with condensation are significantly longer than those associated with the detection of a medicament release. Alternatively or additionally, a threshold level for reliable detection of a medicament release may be adjusted automatically in dependence on a baseline value. In such circumstances, the micro-controller may be configured to interpret a drop in the detector output signal of a predetermined percentage of the baseline signal as indicative of a medicament release.

Continuous compensation for ambient light can conveniently be made by taking two measurements from the detector for every current pulse supplied to the emitter. In such circumstances, It is convenlent to take one detector measurement with the emitter inactive and another with the emitter operative, and subtracting the inactive reading from the operative one.

In any event it is convenient for the sensor operation to be monitored as part of a final test procedure during manufacture. In this operation, infra red radiation, modulated to simulate interaction of a beam of infra red energy with a medicament release of correct dosage, is caused to impinge on the detector, and the resulting output signals are checked, as to magnitude, profile and timing, to ensure that the sensor and associated electronic components are capable of operating correctly to detect delivery of a correct dose of the medicament.

Means for resisting deposition of contaminants may be provided at locations capable of adversely affecting the detection of said release by said sensor. Typically, such locations will comprise a surface of the emitter and/or its associated detector, or any window or light pipe or other optical element associated with either the emitter or the detector and having a surface exposed to the medicament release, or other surfaces closely proximate to any of the foregoing.

In one example, the means for resisting deposition of contaminants comprises the application, by any convenient method, of non-adhesive coatings of materials such as Teflon (Registered Trade Mark) or other fluoropolymer materials which reduce the tendency of chemical to adhere thereto and/or anti-static agents, hydrophobic or hydrophilic materials directly to the surfaces at risk.

In alternative examples, baffles, bluff bodies or other such physical barriers or guides are disposed and/or configured to obstruct the passage of contaminants toward locations at risk, and/or to positively divert contaminants away from such locations,

In other examples, suitably placed and configured air inlets are provided on or near the mouthpiece to permit clean air to be drawn past locations at risk of contamination, when the dispenser is used.

it will also be appreciated that any combination of any two or more of the foregoing expedients may be used if desired or necessary.

In this respect, Figure 10 shows the use of both baffles and air flow to reduce powder deposition on surfaces in the path of the infra red beam. In Figure 10, infra red radiation from an emitter (not shown) emerges from a surface 693 of a light guide 691, traverses an outlet 650 of the dispenser and enters a surface 697 of a light guide 695 that leads to a detector (not shown). A mouthpiece (not shown) associated with the outlet is disposed to the left in the drawing, so that the medicament release flows from right to left when the patient inhales. Baffles 683 and 687 are placed upstream of the surfaces 693 and 697 respectively to divert the medicament release flow away from the surfaces 693 and 697; the diversion being assisted by air, drawn in through vents 698 and 699 when the patient inhales; the vents being disposed near the baffles and just upstream of the surfaces 693 and 697 respectively.

In practice, the baffles 683 and 687 may not be formed as individual elements but may instead form part of an annular baffle configuration that may comprise one or more rings, preferably slanted with respect to the medicament release flow. A spider arrangement may usefully be employed to hold a multi-ringed baffle in place. Moreover, the vents 698 and 699 may take any form or configuration that assists either to keep contaminants away from the surfaces 693 and 697 or in removing, as by blowing away, contaminant that might find its way on to either surface.

Means may be provided for removing, at least in part, contaminants deposited at locations capable of adversely affecting the detection of the medicament release.

The use of ducted air, sucked through vents when the patient inhales, in this respect has already been discussed.

in another example, particularly applicable to contaminant in the form of moisture condensing on surfaces in the path of the radiation beam as a result of the use of the mouthpiece by a patient, the means for removing comprises means for applying heat to the said locations.

In such an example, it is preferred that microwires or a resistive layer are deposited on any surface at risk to form local heaters which can be operated when necessary to drive off the condensation, for example by evaporation. The local heaters are operated, under control of the microprocessor, only when it is specifically necessary, and for a minimised time, in order to reduce the drain on the power supply.

In another example any surface at risk of contamination can be coated or otherwise treated to encourage absorption of a proportion of the energy contained in the infra-red beam, or other beam of energy, used in the sensing process or in another beam used in temporary substitution for such a beam.

In such circumstances, where another beam of radiation is temporarily substituted for the beam or beams used in the sensing process, the substitute beam is conveniently chosen to be of a different form, or of a different wavelength, from the beam or beams used in the sensing process. This permits the relevant surface at risk to be treated with a material that absorbs the radiation of the substitute beam to a useful extent, but does not significantly absorb the beam or beams used in the sensing process, thus allowing the sensitivity of the sensing process to be maintained.

Where a substituted beam is used for the above purpose, it may be automatically generated at predetermined or random intervals that are preferably timed and/or controlled so as not to coincide with the emission of the beam or beams used in the sensing process. Alternatively, and preferably, however, the substitute beam is generated, as part of a cleansing operation, only when calibration readings derived from the sensing process indicate that contaminants have built up to an extent that requires cleaning.

In another example of this embodiment of the invention, agitating components are provided at or adjacent any surface at risk of contamination by the medicament release.

Where the means for removing contaminants involves agitation, a preferred procedure Is to provide a transducer of piezoelectric or magnetostrictive material at or near a surface susceptible of contamination, and to apply electrical oscillation signals to the transducer so as to cause it to vibrate when it is desired to decontaminate the surface. Preferably, the vibration Is caused to occur at a selected frequency, such as a resonant frequency of the transducer, or at a range of frequencies intended to promote the release of contaminants from the surface in question.

The piezoelectric or magnetostrictive material is, in this example, annular and placed so as to directly surround at least part of the surface, thereby to allow physical vibrations to be generated In extremely close proximity to said surface without interfering with the sensing process.

In another example, particularly appropriate where piezoelectric material is used, the transducer is deposited, or mounted as a separate entity, directly on top of the surface to be cleansed, provided that the material of which the transducer is made does not unduly absorb the beam or beams used in the sensing process.

In a further alternative embodiment, mechanically movable or electrically driven scraper devices are incorporated into the dispenser to enable contaminants to be physically scraped away from surfaces at risk.

It will be understood that the present disclosure is for the purpose of illustration only and the invention extends to modifications, variations and improvements thereto within the scope of the claims, and it is again stressed that the present invention is applicable to any of the forms of medicament dispenser described or referred to hereinbefore; in particular it is applicable to dispensers of the dry powder or the aerosol kind.

## Claims

1. A medicament dispenser comprising:
(i) a housing (10; 710) having an outlet (550),
(ii) a medicament container (20;720) locatable within said housing;
(iii) an electronic dose counter (70) associated with said outlet; wherein said dose counter comprises a first sensor (560, 561; 660, 662) for directly detecting a medicament release dispensable from said medicament container through said outlet; **characterised in that** the medicament dispenses further comprises
(iv) means (620) for detecting changes in the performance of the sensor attributable to contamination or degradation and for adjusting the operation of the dispenser to compensate, at least In part, for said changes.

2. A dispenser according to claim 1 wherein said means for detecting changes in the performance of the sensor comprises electronic means (620), utilising the sensor, for taking a calibration reading prior to the dispensing of a dose of medicament.

3. A dispenser according to claim 2 further comprising means for comparing the calibration reading with a predetermined threshold value indicative of satisfactory operation of the sensor and for changing an operating characteristic of the sensor and/or of one or more electronic components associated therewith, in the event that the comparison indicates a change from said satisfactory operation, in a sense tending to restore satisfactory operation of the sensor.

4. A dispenser according to claim 3 wherein the sensor includes an emitter (560;660) and a detector (561;661) of a beam of radiation, such as infra red radiation, and the said operating characteristic comprises the magnitude of driving current pulses applied to the emitter.

5. A dispenser according to claim 4 including means for deriving said calibration reading from electrical signals derived from the detector In response to radiation emitted by the emitter in response to said current pulses.

6. A dispenser according to claim 4 or claim 5 including means for progressively increasing the magnitude of said current from a starting level until said calibration reading matches said threshold level.

7. A dispenser according to claim 6, wherein the means for progressively increasing the magnitude of said current effects said increase step-wise.

8. A dispenser according to claim 7 wherein the steps are substantially equal in magnitude.

9. A dispenser according to claim 2 or claim 3 wherein said electronic means comprises part of a servo system that directly compensates for changes in performance as they occur.

10. A dispenser according to any of claims 2 to 9 wherein said electronic means includes a microprocessor (620).

11. A medicament dispenser according to any preceding claim, wherein said sensor comprises an emitter (560; 660) and a detector (561;661).

12. A medicament dispenser according to claim 11, wherein the emitter emits electromagnetic radiation.

13. A medicament dispenser according to claim 11 or claim 12, wherein the detector detects electromagnetic radiation.

14. A medicament dispenser according to claim 12 or claim 13 as dependent on claim 12, wherein the electromagnetic radiation emitted from the emitter is infrared, visible or ultraviolet radiation.

15. A medicament dispenser according to claim 14, wherein the radiation is in the range of 0.95µm to 0.35µm or has a wavelength peak at about 0.88µm.

16. A medicament dispenser according to any one of claims 11 to 15, wherein the emitter is selected from the group consisting of light emitting diode, laser, incandescent lamp, electroluminescent or fluorescent light sources.

17. A medicament dispenser according to claim 16, wherein the emitter further comprises a filter, preferably an optical filter and more preferably an optical filter which is a polarising filter.

18. A medicament dispenser according to any one of claims 11 to 17, wherein the detector is selected from the group consisting of photodiode, phototransistor, light dependent resistor, pyroelectric detector and bolometer.

19. A medicament dispenser according to claim 18, wherein the detector further comprises a filter, preferably an electronic filter, more preferably an optical filter and yet more preferably an optical filter which is a polarising filter.

20. A medicament dispenser according to any one of claims 11 to 19, wherein the detector is associated with an amplifler.

21. A medicament dispenser according to claim 20, wherein the amplifier is positioned close to the detector and/or the amplifier is integrated with the detector.

22. A medicament dispenser according to any one of claims 11 to 21, wherein the detector detects an increase or decrease in radiation compared to the amount of radiation emitted by the emitter.

23. A medicament dispenser according to claim 22 wherein the increases or decrease in detected radiation is due to Interference of radiation reaching the detector by the medicament release.

24. A medicament dispenser according to any one of claims 11 to 23, wherein the amount of radiation reaching the detector is maintained at a substantially constant level by using an electronic feedback circuit to alter the level of radiation emitted by the emitter.

25. A medicament dispenser according to any one of claims 11 to 24, wherein the first sensor further comprises a reflector to reflect radiation from the emitter to the detector.

26. A medicament dispenser according to any one of claims 11 to 25, wherein the emitter emits radiation of more than one wavelength and the detector detects radiation of more than one wavelength.

27. A medicament dispenser according to claim 26, wherein the first sensor quantifies the concentration of medicament with the medicament release by measuring radiation at one or more wavelengths.

28. A medicament dispenser according to any one of claims 11 to 27, wherein the dispenser additionally comprises a second sensor for detecting a medicament release.

29. A medicament dispenser according to claim 28, wherein the second sensor comprises an emitter and a detector.

30. A medicament dispenser according to claim 28 or claim 29, wherein the medicament release passes the second sensor subsequent to passing the first sensor.

31. A medicament dispenser according to any one of claims 11 to 30, wherein the dispenser further comprises a third sensor.

32. A medicament dispenser according to claim 31, wherein the third sensor is sensitive to a parameter selected from the group consisting of electromagnetic radiation, magnetic field, electric field, light, motion, temperature, pressure, sound, oxygen concentration, carbon dioxide concentration and moisture.

33. A medicament dispenser according to claim 31 or claim 32, wherein the third sensor responds to actuation of the dispenser.

34. A medicament dispenser according to any preceding claim, wherein the first sensor is integral with the outlet.

35. A medicament dispenser according to claim 28 or to any claim appended thereto, wherein the second sensor is integral with the outlet.

36. A medicament dispenser according to any preceding claim, wherein the medicament container is an aerosol contained (10; 710).

37. A medicament dispenser according to any one of claims 1 to 35, wherein the medicament container is a dry-powder container.

38. A medicament dispenser according to any preceding claim wherein the outlet comprises a mouthpiece (551) for inhalation therethrough.

## Patentansprüche

1. Medikamentenspender, mit:
(i) einem Gehäuse (10; 710), das einen Auslass (550) aufweist;
(ii) einem Medikamentenbehälter (20; 720), der innerhalb des Gehäuses unterbringbar ist;
(iii) einem elektronischen Dosiszähler (70), der mit den Auslass verbunden ist; wobei der Dosiszähler einen ersten Sensor (560, 561; 660, 661) umfasst, zum direkten Erfassen einer Medikamentenfreigabe, die aus dem Medikamentenbehälter durch den Auslass abgebbar ist; **dadurch gekennzeichnet, dass** der Medikamentenspender ferner umfasst:
(iv) eine Einrichtung (620) zum Erfassen von Änderungen beim Betriebsverhalten des Sensors, die einer Verunreinigung oder Verschlechterung zuschreibbar sind, und zum Anpassen des Betriebs des Spenders, um zumindest teilweise die Änderungen zu kompensieren.

2. Spender nach Anspruch 1, bei dem die Einrichtung zum Erfassen von Änderungen beim Betriebsverhalten des Sensors eine elektronische Einrichtung (620) umfasst, die den Sensor benutzt, zum Vornehmen einer Kalibrierablesung vor dem Abgeben einer Medikamentendosis.

3. Spender nach Anspruch 2, ferner mit einer Einrichtung zum Vergleichen der Kalibrierablesung mit einem vorbestimmten Schwellenwert, der auf einen zufriedenstellenden Betrieb des Sensors hindeutet, und zum Ändern einer Betriebscharakteristik des Sensors und/oder von einer oder mehreren damit verbundenen elektronischen Komponenten, in dem Fall, dass der Vergleich auf eine Änderung von dem zufriedenstellenden Betrieb hindeutet, in einem Sinn, abzielend auf ein Wiederherstellen eines zufriedenstellenden Betriebs des Sensors.

4. Spender nach Anspruch 3, bei dem der Sensor einen Emitter (560; 660) und einen Detektor (561; 661) eines Strahls von Strahlung umfasst, wie beispielsweise Infrarotstrahlung, und wobei die Betriebscharakteristik die Größenordnung eines Antriebsstromimpulses umfasst, der an den Emitter angelegt wird.

5. Spender nach Anspruch 4, mit einer Einrichtung zum Ableiten der Kalibrierablesung aus elektrischen Signalen, die von dem Detektor stammen, als Reaktion auf durch den Emitter, als Reaktion auf die Stromimpulse, abgegebene Strahlung.

6. Spender nach Anspruch 4 oder 5, mit einer Einrichtung zum progressiven Erhöhen der Größenordnung des Stroms von einem Anfangspegel, bis die Kalibrierablesung mit dem Schwellenpegel übereinstimmt.

7. Spender nach Anspruch 6, bei dem die Einrichtung zum progressiven Erhöhen der Größenordnung des Stroms die Erhöhung schrittweise bewirkt.

8. Spender nach Anspruch 7, bei dem die Schritte im Wesentlichen gleich in der Größenordnung sind.

9. Spender nach Anspruch 2 oder Anspruch 3, bei dem die elektronische Einrichtung Teil eines Servosystems umfasst, das Änderungen beim Betriebsverhalten direkt wenn sie auftreten kompensiert.

10. Spender nach einem der Ansprüche 2 bis 9, bei dem die elektronische Einrichtung einen Mikroprozessor (620) umfasst.

11. Medikamentenspender nach einem der vorhergehenden Ansprüche, bei dem der Sensor einen Emitter (560; 660) und einen Detektor (561; 661) umfasst.

12. Medikamentenspender nach Anspruch 11, bei dem der Emitter elektromagnetische Strahlung abgibt.

13. Medikamentenspender nach Anspruch 11 oder Anspruch 12, bei dem der Detektor elektromagnetische Strahlung erfasst.

14. Medikamentenspender nach Anspruch 12 oder Anspruch 13, bei Abhängigkeit von Anspruch 12, bei dem die von dem Emitter abgegebene elektromagnetische Strahlung Infrarotstrahlung, sichtbare Strahlung oder Ultraviolettstrahlung ist.

15. Medikamentenspender nach Anspruch 14, bei dem die Strahlung in dem Bereich von 0,95µm bis 0,35µm liegt oder einen Wellenlängenpeak bei ungefähr 0,88µm aufweist.

16. Medikamentenspender nach einem der Ansprüche 11 bis 15, bei dem der Emitter aus der Gruppe ausgewählt wird, die aus Leuchtdiode, Laser, Glühlampe, elektroluminiszenten Lichtquellen oder Fluoreszenzlichtquellen besteht.

17. Medikamentenspender nach Anspruch 16, bei dem der Emitter ferner einen Filter umfasst, vorzugsweise einen optischen Filter, und bevorzugter einen optischen Filter, der ein Polarisationsfilter ist.

18. Medikamentenspender nach einem der Ansprüche 11 bis 17, bei dem der Detektor aus der Gruppe ausgewählt ist, die aus Photodiode, Phototransistor, lichtabhängigem Widerstand, pyroelektrischem Detektor und Bolometer besteht.

19. Medikamentenspender nach Anspruch 18, bei dem der Detektor ferner einen Filter umfasst, vorzugsweise einen optischen Filter, und noch bevorzugter einen optischen Filter, der ein Polarisationsfilter ist.

20. Medikamentenspender nach einem der Ansprüche 11 bis 19, bei dem der Detektor mit einem Verstärker verbunden ist.

21. Medikamentenspender nach Anspruch 20, bei dem der Verstärker nahe an dem Detektor positioniert ist und/oder der Verstärker mit dem Detektor integriert ist.

22. Medikamentenspender nach einem der Ansprüche 11 bis 21, bei dem der Detektor eine Zunahme oder Abnahme der Strahlung erfasst, verglichen mit der durch den Emitter abgegebenen Strahlungsmenge.

23. Medikamentenspender nach Anspruch 22, bei dem die Zunahme oder Abnahme der erfassten Strahlung durch Strahlungsinterferenz verursacht wird, die den Detektor durch die Medikamentenfreigabe erreicht.

24. Medikamentenspender nach einem der Ansprüche 11 bis 23, bei dem die Strahlungsmenge, die den Detektor erreicht, bei einem im Wesentlichen konstanten Pegel beibehalten wird, durch Verwendung einer elektronischen Rückkopplungsschaltung, um den durch den Emitter abgegebenen Strahlungspegel zu verändern.

25. Medikamentenspender nach einem der Ansprüche 11 bis 24, bei dem der erste Sensor ferner einen Reflektor umfasst, um Strahlung von dem Emitter zu dem Detektor zu reflektieren.

26. Medikamentenspender nach einem der Ansprüche 11 bis 25, bei dem der Emitter Strahlung von mehr als einer Wellenlänge abgibt und der Detektor Strahlung von mehr als einer Wellenlänge erfasst.

27. Medikamentenspender nach Anspruch 26, bei dem der erste Sensor die Medikamentenkonzentration bei der Medikamentenfreigabe quantifiziert, durch Messen von Strahlung bei einer oder mehreren Wellenlängen.

28. Medikamentenspender nach einem der Ansprüche 11 bis 27, bei dem der Spender außerdem einen zweiten Sensor zum Erfassen einer Medikamentenfreigabe umfasst.

29. Medikamentenspender nach Anspruch 28, bei dem der zweite Sensor einen Emitter und einen Detektor umfasst.

30. Medikamentenspender nach Anspruch 28 oder Anspruch 29, bei dem die Medikamentenfreigabe an dem zweiten Sensor, nach einem Vorbeigehen an dem ersten Sensor, vorbeigeht.

31. Medikamentenspender nach einem der Ansprüche 11 bis 30, bei dem der Spender ferner einen dritten Sensor umfasst.

32. Medikamentenspender nach Anspruch 31, bei dem der dritte Sensor auf einen Parameter empfindlich reagiert, der aus der Gruppe ausgewählt ist, die aus elektromagnetischer Strahlung, Magnetfeld, elektrisches Feld, Licht, Bewegung, Temperatur, Druck, Schall, Sauerstoffkonzentration, Kohlendioxidkonzentration und Feuchtigkeit besteht.

33. Medikamentenspender nach Anspruch 31 oder Anspruch 32, bei dem der dritte Sensor auf eine Betätigung des Spenders reagiert.

34. Medikamentenspender nach einem der vorhergehenden Ansprüche, bei dem der erste Sensor mit dem Auslass integriert ist.

35. Medikamentenspender nach Anspruch 28, oder einem davon abhängigen Anspruch, bei dem der zweite Sensor mit dem Auslass integriert ist.

36. Medikamentenspender nach einem der vorhergehenden Ansprüche, bei dem der Medikamentenbehälter ein AerosolBehälter (10; 710) ist.

37. Medikamentenspender nach einem der Ansprüche 1 bis 35, bei dem der Medikamentenbehälter ein Trockenpulverbehälter ist.

38. Medikamentenspender nach einem der vorhergehenden Ansprüche, bei dem der Auslass ein Mundstück (551) zur Inhalation **dadurch** umfasst.

## Revendications

1. Distributeur de médicament comprenant :
(i) un boîtier (10 ; 710), ayant une sortie (550) ;
(ii) un conteneur de médicament (20 ; 720) pouvant être situé à l'intérieur dudit boîtier ;
(iii) un compteur de dose électronique (70) associé à ladite sortie ; dans lequel ledit compteur de dose comprend un premier capteur (560, 561 ; 660, 661) pour détecter directement une libération de médicament distribuable à partir dudit conteneur de médicament à travers ladite sortie ; **caractérisé en ce que** le distributeur de médicament comprend en outre
(iv) des moyens (620) pour détecter des changements de performances du capteur attribuables à une contamination ou à une dégradation et pour ajuster le fonctionnement du distributeur pour compenser, au moins en partie, lesdits changements.

2. Distributeur selon la revendication 1, dans lequel lesdits moyens pour détecter des changements dans les performances du capteur comprennent des moyens électroniques (620), utilisant le capteur, pour prendre un relevé d'étalonnage avant la distribution d'une dose de médicament.

3. Distributeur selon la revendication 2, comprenant en outre des moyens pour comparer le relevé d'étalonnage à une valeur de seuil prédéterminée indicatrice d'un fonctionnement satisfaisant du capteur et pour changer une caractéristique de fonctionnement du capteur et/ou d'un ou plusieurs composants électroniques associés à celui-ci, dans le cas où la comparaison indique un changement par rapport audit fonctionnement satisfaisant, dans un sens tendant à rétablir le fonctionnement satisfaisant du capteur.

4. Distributeur selon la revendication 3, dans lequel le capteur comprend un émetteur (560 ; 660) et un détecteur (561 ; 661) d'un faisceau de rayonnement, comme un rayonnement infrarouge, et ladite caractéristique de fonctionnement comprend la grandeur des impulsions de courant d'entraînement appliquées à l'émetteur.

5. Distributeur selon la revendication 4, comprenant des moyens pour dériver ledit relevé d'étalonnage de signaux électriques dérivés du détecteur en réponse au rayonnement émis par l'émetteur en réponse auxdites impulsions de courant.

6. Distributeur selon la revendication 4 ou la revendication 5, comprenant des moyens pour augmenter progressivement la grandeur dudit courant à partir d'un niveau de départ jusqu'à ce que ledit relevé d'étalonnage atteigne ledit niveau de seuil.

7. Distributeur selon la revendication 6, dans lequel les moyens pour augmenter progressivement la grandeur dudit courant effectuent ladite augmentation par incréments.

8. Distributeur selon la revendication 7, dans lequel les incréments sont sensiblement de grandeur égale.

9. Distributeur selon la revendication 2 ou la revendication 3, dans lequel lesdits moyens électroniques comprennent une partie d'un système d'asservissement qui compense directement les changements de performances lorsqu'ils interviennent.

10. Distributeur selon l'une quelconque des revendications 2 à 9, dans lequel lesdits moyens électroniques comprennent un microprocesseur (620).

11. Distributeur de médicament selon l'une quelconque des revendications précédentes, dans lequel ledit capteur comprend un émetteur (560 ; 660) et un détecteur (561 ; 661).

12. Distributeur de médicament selon la revendication 11, dans lequel l'émetteur émet un rayonnement électromagnétique.

13. Distributeur de médicament selon la revendication 11 ou la revendication 12, dans lequel le détecteur détecte un rayonnement électromagnétique.

14. Distributeur de médicament selon la revendication 12 ou la revendication 13 dépendant de la revendication 12, dans lequel le rayonnement électromagnétique émis de l'émetteur est un rayonnement infrarouge, visible ou ultraviolet.

15. Distributeur de médicament selon la revendication 14, dans lequel le rayonnement se trouve dans la plage de 0,95 µm à 0,35 µm ou possède une crête de longueur d'onde à environ 0,88 µm.

16. Distributeur de médicament selon l'une quelconque des revendications 11 à 15, dans lequel l'émetteur est sélectionné dans le groupe se composant d'une diode d'émission de lumière, d'un laser, d'une lampe incandescente, d'une source de lumière électroluminescente ou d'une source de lumière fluorescente.

17. Distributeur de médicament selon la revendication 16, dans lequel l'émetteur comprend en outre un filtre, de préférence un filtre optique, et, de manière davantage préférée, un filtre optique qui est un filtre de polarisation.

18. Distributeur de médicament selon l'une quelconque des revendications 11 à 17, dans lequel le détecteur est sélectionné dans le groupe se composant d'une photodiode, d'un phototransistor, d'une résistance dépendant de la lumière, d'un détecteur pyroélectrique et d'un bolomètre.

19. Distributeur de médicament selon la revendication 18, dans lequel le détecteur comprend en outre un filtre, de préférence un filtre électronique, de manière davantage préférée un filtre optique et de manière encore davantage préférée un filtre optique qui est un filtre polarisant.

20. Distributeur de médicament selon l'une quelconque des revendications 11 à 19, dans lequel le détecteur est associé à un amplificateur.

21. Distributeur de médicament selon la revendication 20, dans lequel l'amplificateur est positionné à proximité du détecteur et/ou l'amplificateur est intégré au détecteur.

22. Distributeur de médicament selon l'une quelconque des revendications 11 à 21, dans lequel le détecteur détecte une augmentation ou une réduction du rayonnement par comparaison à la quantité de rayonnement émis par l'émetteur.

23. Distributeur de médicament selon la revendication 22, dans lequel l'augmentation ou la réduction du rayonnement détecté est due à l'interférence du rayonnement atteignant le détecteur par la libération de médicament.

24. Distributeur de médicament selon l'une quelconque des revendications 11 à 23, dans lequel la quantité de rayonnement atteignant le détecteur est maintenue à un niveau sensiblement constant en utilisant un circuit de retour électronique pour altérer le niveau de rayonnement émis par l'émetteur.

25. Distributeur de médicament selon l'une quelconque des revendications 11 à 24, dans lequel le premier capteur comprend en outre un réflecteur pour refléter le rayonnement de l'émetteur vers le détecteur.

26. Distributeur de médicament selon l'une quelconque des revendications 11 à 25, dans lequel l'émetteur émet un rayonnement de plus d'une longueur d'onde et le détecteur détecte un rayonnement de plus d'une longueur d'onde.

27. Distributeur de médicament selon la revendication 26, dans lequel le premier capteur quantifie la concentration de médicament avec la libération de médicament en mesurant le rayonnement à une ou plusieurs longueurs d'onde.

28. Distributeur de médicament selon l'une quelconque des revendications 11 à 27, dans lequel le distributeur comprend en outre un deuxième capteur pour détecter une libération de médicament.

29. Distributeur de médicament selon la revendication 28, dans lequel le deuxième capteur comprend un émetteur et un détecteur.

30. Distributeur de médicament selon la revendication 28 ou la revendication 29, dans lequel la libération de médicament passe par le deuxième capteur après être passée par le premier capteur.

31. Distributeur de médicament selon l'une quelconque des revendications 11 à 30, dans lequel le distributeur comprend en outre un troisième capteur.

32. Distributeur de médicament selon la revendication 31, dans lequel le troisième capteur est sensible à un paramètre sélectionné dans le groupe se composant d'un rayonnement électromagnétique, d'un champ magnétique, d'un champ électrique, d'une lumière, d'un mouvement, d'une température, d'une pression, d'un son, d'une concentration d'oxygène, d'une concentration de dioxyde de carbone et d'une humidité.

33. Distributeur de médicament selon la revendication 31 ou la revendication 32, dans lequel le troisième capteur répond à l'actionnement du distributeur.

34. Distributeur de médicament selon l'une quelconque des revendications précédentes, dans lequel le premier capteur fait partie intégrale de la sortie.

35. Distributeur de médicament selon la revendication 28 ou n'importe quelle revendication dépendante à celle-ci, dans lequel le deuxième capteur fait partie intégrale de la sortie.

36. Distributeur de médicament selon l'une quelconque des revendications précédentes, dans lequel le conteneur de médicament est un conteneur d'aérosol (10 ; 710).

37. Distributeur de médicament selon l'une quelconque des revendications 1 à 35, dans lequel le conteneur de médicament est un conteneur de poudre sèche.

38. Distributeur de médicament selon l'une quelconque des revendications précédentes, dans lequel la sortie comprend un embout buccal (551) pour l'inhalation à travers celui-ci.
